(19) Europäisches Patentamt
European Patent Office
Office européen des brevets

(11) Publication number: **0 152 886 A2**

# (12) EUROPEAN PATENT APPLICATION

(21) Application number: 85101407.6

(22) Date of filing: 11.02.85

(51) Int. Cl.[4]: **G 01 N 33/543**, C 12 Q 1/68 // G01N33/548

(30) Priority: 22.02.84 US 582503

(43) Date of publication of application: 28.08.85 Bulletin 85/35

(84) Designated Contracting States: CH DE FR GB IT LI NL SE

(71) Applicant: **Molecular Diagnostics, Inc., 400 Morgan Lane, West Haven, CT.06516 (US)**

(72) Inventor: **Dattagupta, Nanibhushan, 470 Prospect Street, New Haven, Ct 06511 (US)**

(74) Representative: **Adrian, Albert, Dr. et al, c/o BAYER AG Konzernverwaltung RP Patentabteilung, D-5090 Leverkusen 1, Bayerwerk (DE)**

## (54) Immobilized nucleic acid-containing probes.

(57) An immobilized nucleic acid-containing probe suitable for accurate hybridization gene analyses is produced by enzymatically coupling a nucleotide to a predetermined end of the nucleic acid and then selectively coupling a suitably prepared solid support, such as cellulose, through the nucleotide. Thus the ribose of the nucleotide can be oxidized by sodium periodate to form aldehyde groups which will react with a primary amino group introduced into collulose, forming a Schiff's base. For grater stability of the immobilizing bond the Schiff's base is reduced to a saturated secondary amino group by reduction with borohydride. Alternatively, the nucleotide may carry an SH or Hg moiety which will selectively react with complementary moieties on the solid support.

EP 0 152 886 A2

0152886

The invention relates to an improved way of coupling a nucleic acid-containing probe to a solid support.

In European Patent Application No. 84 107 248.1 there is disclosed a procedure for testing samples for the presence or absence therein of particular nucleic acid sequences, as in the diagnosis of certain hereditary conditions. The particular technique is called dual hybridization and involves two testing components, each including a nucleic acid moiety. One of the components carries a label and is the detection probe, reading of its label being the ultimate step in making the assay. The other component has its nucleic acid attached to a solid support, i.e., is immobilized, and it is known as the separation probe.

In other systems it is sometimes the unknown which is immobilized.

The ultimate success and efficiency of the assay technique of European Patent Application No. 84 107 248.1 depend upon the proper attachment and labeling of the separation and detection probes. If the coupling of the separation probe is done via the 5' end, the detection probe should be labeled at the 3' end to avoid the presence of the label in the middle of the hybrid. Although all orientations will work, the most effective and desirable orientations should be unidirectional with respect to the end of the DNA.

The existing methods used to attach a poly-nucleotide probe to a solid support are non-specific and the number of attachment sites per nucleic acid is difficult to control. It has been found that multiple attachment reduces the degree of freedom of the

immobilized nucleic acid. The physical adsorption of single stranded DNA, covalent attachment via diazo-linkage, epoxidation, cyanogen bromide activation and photochemical reactions are associated with the complication of non-specific linkage between the nucleic acids and the solid support.

It is accordingly an object of the invention to provide a method of coupling a nucleic acid to a solid support specifically, rather than non-specifically.

This and other objects and advantages are realized in accordance with the present invention pursuant to which a nucleic acid probe is coupled to a solid support in a manner which is site specific, which does not interfere with the ability of the nucleic acid to hybridize and which involves preferably a single chemical covalent linkage per nucleic acid to the solid support.

Specifically, a nucleotide is coupled to the nucleic acid employing an enzyme and the nucleotide is chemically modified. Independently, the solid support is modified chemically to ensure the presence of groups which are specifically reactive with the chemically modified nucleotide fragment. Then the two materials are reacted, the nucleic acid becoming chemically bonded to the support through the nucleotide fragment, leaving the nucleic acid free for hybridization.

The nucleic acid material employed in the probe can be any of those known, e.g. DNA, RNA, an oligonucleotide, etc.

The material coupled to the nucleic acid can be a nucleotide containing hydroxy groups in the 2,3-positions or a nucleotide carrying a Hg, S or similar

group which can thereafter react specifically with the properly prepared solid substrate.

The solid support can be of any composition, onto which selectively reactive functional groups have been introduced. Thus, nylon, acrylics, Sephadex, Sepharose, and the like may be employed, but excellent results are achieved with hydroxy-containing materials such as cellulose and cellulose esters such as cellulose nitrate, cellulose acetate, and the like.

The invention will be further described with reference to the accompanying drawings, wherein:

Fig. 1 is a schematic flow sheet of an embodiment in accordance with the invention of coupling DNA to a solid support employing a ribonucleotide; and

Fig. 2 is a schematic flow sheet of several embodiments of coupling DNA to a solid support utilizing a modified nucleotide.

Referring now more particularly to the drawings, in Fig. 1 a ribonucleotide (r) is enzymatically coupled to a predetermined end, e.g. the 3' end, of the nucleic acid (NA) desired in the end product. By means of sodium periodate there takes place a cleavage of the ribose ring between the 2- and 3-positions (cis-diol), a reaction for which periodate is specific. The hydroxyl groups are converted to aldehyde groups so the nucleic acid moiety now carries two aldehyde groups at its 3'-end.

In a known manner, a solid support is provided with specifically reactive sites, e.g. $NH_2$, SH, Hg, or the like. In Fig. 1 the site is $NH_2$. The solid support carrying the $NH_2$ groups is then reacted with the CHO groups attached to the end of the nucleic acid,

forming what is known as a Schiff's base having a -CH=N- configuration. Since this is somewhat unstable it is converted to the more stable saturated $—CH_2$-HN- form by selective reduction as with a borohydride.

If the starting nucleic acid already contains a ribose moiety it is not necessary to effect the initial enzymatic coupling.

In this figure a single amine has been shown as reacting with a single aldehyde radical but conceivably a single amine could react with two aldehyde radicals ultimately leading to a -CH-NR-CH- configuration.

In Fig. 2 one again starts with a nucleic acid (NA) probe to one end of which a nucleotide is enzymatically coupled. However, the nucleotide has a modified structure on its heterocyclic ring (mod-N'), i.e. the purine or pyrimidine ring carries a SH or Hg radical.

In the left-most branch the ring carries a Hg radical and is reacted with a solid support carrying a SH radical, produced by any suitable method.

In the middle and right embodiments the mod-N' carries a SH moiety which reacts with a complementary reactive moiety on the solid support. This may be Hg as in the middle branch or it may be residue of N-succinimidyl-3-(2-pyridyldithio)-propionate (SPDP) as at the right. The end product in the right branch thus has a dithio -S-S- couple while the middle and left branches have a -Hg-S- couple, in either direction.

The individual steps of the several reactions are known in the art with regard to stoichiometry and reaction conditions. Other selective couplings involving

other reactive groups will also suggest themselves to those skilled in the art, e.g. epoxy groups, dienes, azo groups, and the like.

The invention will be further described in the following illustrative examples wherein all parts are by weight unless otherwise expressed.

## Example 1

Preparation of the solid support containing Amine ($NH_2$) groups.

Cellulose powder (or paper) (0.5 g) (Cellex NI, Bio-Rad) is mixed (soaked) with 5 ml. distilled water at room temperature for approximately 30 minutes. The cellulose product is washed with distilled water and resuspended in 20 ml ice cold distilled water. The suspension is adjusted to pH 10.5-11 with a 5 M solution of sodium hydroxide. The suspension is cooled in an ice-bath (4° C) and 1.0 g solid cyanogen bromide is added to the suspension. The suspension is retained in the ice-bath and continuously stirred for 30 minutes. During this period, the pH is continuously monitored and maintained at 10.5-11 with sodium hydroxide (5 M). The suspension is washed with ice cold distilled water and treated with approximately 5 ml of hexamethylene diamine (1 M solution in $H_2O$, adjusted to pH 9-10 with HCl). The cellulose amine product is washed with water or other suitable buffer, depending on the future use of the product.

## Example 2

Preparation of N-succinimidyl 3-(2-pyridyldithio)-propionate (SPDP) activated solid support.

Cellulose amine solid support is prepared as described above (see Example 1). SPDP is dissolved in

ethanol to obtain a 20 mM stock solution. The cellulose amine is suspended in 5 ml potassium phosphate buffer (10 mM, pH 8) to which 100 µl of the SPDP stock solution is added. The reaction is allowed to proceed for six hours in accordance with the technique described by J. Carlsson, H. Drevin & R. Axen, Biochem. J., 173, 723-737, (1978) To eliminate the uncoupled SPDP, the suspension is centrifuged at 2000 g, 25° C, for 5 minutes, the supernatant discarded and the pellet resuspended in 5 ml of the potassium phosphate buffer. This wash is repeated 3 times. The extent of coupling is determined by the release of pyridine-2-thione upon treatment of an aliquot of the suspension with dithiothreitol (final concentration 20 mM). The amount of pyridine-2-thione released is estimated from the optical density [molar absorbancy index at 343 nm. ($8.08 \times 10^3$) $M^{-1}$ $cm^{-1}$] (Stuchbury et al, Biochem. J., 151, pg. 417, 1975).

Example 3

Preparation of solid support with reactive thiol residues.

a) Cellulose amine solid support is prepared as described above (see Example 1). Approximately 1 g wet cellulose amine is suspended in 5 ml $NaHCO_3$ buffer, 0.1 M, pH 9.7. N-acetyl-homocysteine thiolactone (0.2 g) is added. The suspension is stirred for 24 hours at room temperature (25° C).

b) In accordance with the procedures for preparing sulfhydryl agarose, as described by Cuatrecasas et al, J. Biol. Chem., 245, 3059 (1970), a similar procedure is employed to prepare solid support containing -SH residues. Cellulose powder (Cellex NI, Bio Red) is mixed with distilled water (10 g in 50 ml water) at room temperature. The product is activated with cyanogen bromide 250 mg/ml of the suspension. The pH is adjusted

to 11 ± .1 with a 5 M solution of sodium hydroxide. The suspension is cooled to 15° C by immersion in an ice-bath for approximately 30 minutes. The suspension is washed with sodium hydroxide, (1m M, pH 10).

Ethylene diamine (2 M) is added to the suspension in a 1:1 volume ratio and the pH adjusted to 10 with HCl. The suspension is centrifuged (2000 g, 10 minutes) and resuspended in distilled water. The wash is repeated 3 times. The resultant cellulose amine is suspended in 50 ml $NaHCO_3$ buffer, (0.1 M, pH 9.7). N-acetylhomocysteine thiolactone (5 g) is added and the suspension stirred for 24 hours at 4° C.

Since any solid substance containing hydroxy groups can be activated by cyanogen bromide to react with alkyl amines, this procedure can be followed with cellulose paper, Sephadex, Sepharose and agarose.

Example 4

Preparation of solid support containing mercury residues.

Solid support containing mercury residues is prepared in accordance with the procedure described by L. Sluyterman and J. Wijdenes, Biochim. Biophys., Acta 200, 593-595 (1970). Solid support is activated by cyanogen bromide as described above (see Example 1).

The activated cellulose suspension is mixed with 10% DMSO (v/v in water in a 1:1 volume ratio, the pH adjusted to pH 9 and cooled to 0° C for 15 minutes. To the cellulose suspension p-aminophenyl mercurio acetate solution in DMSO is added to a final concentration of 5 mg/ml of the suspension. The reaction is allowed to proceed for 20 hours at 0° C. The mixture is warmed to room temperature and washed with 20% (v/v) DMSO in water

to remove unreacted mercurials.

Example 5

Terminal transferase addition of ribonucleotides, mercurated nucleotides or mercapto nucleotides.

A ribonucleotide or deoxyribonucleotide can be coupled to the 3'-hydroxyl terminus of a DNA molecule or oligonucleotide by the enzymatic reaction of terminal deoxynucleotidyl transferase. Twenty (20) µl DNA (approximately 5 x $10^{-13}$ M) are dissolved in 20 µl potassium cacodylate (1 M, pH 7.2). This solution is incubated with 40 µl distilled water, 5-20 µl dithio threitol (DTT) (2 mM), 1-2 µl nucleotide triphosphate (10 mM) and either 10 µl cobalt chloride (10 mM) in the case of coupling to double-stranded DNA or 50 µl magnesium chloride (20 mM) in the case of coupling to single-stranded DNA or oligonucleotide, at 37° C for 5 minutes. The reaction mixture is subsequently cooled in an ice-bath for 10 minutes. Terminal deoxynucleotidyl transferase (14 - 20 units) is added and the mixture is incubated at 15° for 60 minutes. The modified nucleic acid can be isolated from non-coupled nucleotides and proteins by phenol extraction and alcohol precipitation. If desired, all but one terminal ribonucleotide residue can be digested away with 1 M sodium hydroxide at 40° C. This is to be followed by alkaline phosphatase treatment before oxidation as in Example 6a.

The greater amount of DTT in the 5 - 20 µl range described above is used with the nucleotide triphosphate contains a - SH or -Hg residue. The excess amount of DTT will mask the effect of these residues on the enzymatic activity.

A ribonucleotide or deoxyribonucleotide can be

coupled to the 5' hydroxyl terminus of an oligoribonucleotide by the enzymatic action of T4 RNA ligase in accordance with the method described by Sugino <u>et al</u>, J. Biol. Chem. <u>252</u>, 1732-1738 (1977).

Example 6

Coupling of end labeled nucleic acids to a solid support.

a) Coupling via Schiff's base formation.

It is known that cis-diols can be oxidized by periodate to form dialdehydes. The dialdehydes can form Schiff's base with a primary amine group via the addition of $-NH_2$. The Schiff's base can be reduced with sodium borohydride to form a secondary amine.

In a similar manner, nucleic acids (DNA or RNA) containing ribonucleotides at the 3'-hydroxyl terminus are dissolved in or dialyzed into sodium acetate buffer, 0.1 M, pH 5 at a concentration of 1 mM. Twenty (20) µl of sodium metaperiodate (100 mM) is added to 1 ml of the nucleic acid solution. The reaction is allowed to proceed for 40 minutes at room temperature (25° C). Following the reaction, the pH is adjusted to 8 with sodium hydroxide solution. The solution is added to a cellulose-amine (prepared as described above) suspended in a suitable buffer (conc.) such as 0.1 M sodium acetate, pH 8. The reaction is allowed to proceed for 30 minutes at room temperature (25° C) resulting in the formation of a suspension of Schiff's base. The Schiff's base is reduced by the addition of sodium borohydride. The reduction is carried out in four steps: Approximately 0.15 ml of freshly prepared sodium borohydride solution (200 mM) is added and the reaction is allowed to proceed for 30 minutes. Approximately 0.15 ml of the sodium borohydride solution is again added to

the reaction mixture and the reaction is continued for 60 minutes. Another 0.15 ml of the sodium borohydride is subsequently added to the reaction mixture. After 90 minutes, another aliquot of 0.15 ml of the sodium borohydride solution is added to complete the reaction. The suspension is centrifuged for 30 minutes at 2000 g. The supernatant is decanted and the wash is repeated 3 times. The pellet is resuspended in 5 ml of the hybridization buffer. Etheno ATP or $^{14}C$ ATP residues coupled to single stranded DNA can be used to determine the efficiency of coupling.

b) Coupling of thiol(-SH) containing nucleotide.

Any compound which contains thiol reacts with mercuric or 3-(2-pyridyldithio) propionyl groups. For example, approximately 1 ml of a suspension of SPDP-activated cellulose (prepared as described above in Example 2) in potassium phosphate buffer (10 mM) containing 100 mM sodium chloride is mixed with 1 ml nucleic acid solution containing thiol residues (0.1 mM). The reaction mixture is stirred overnight at room temperature (25° C). The cellulose suspension is centrifuged for 10 minutes at 2000 g. The absorbancy of the supernatant is measured at 343 nm to determine the extent of reaction as compared with a control sample (such as DTT as in Example 2). The cellulose resin is washed with hybridization buffer 3 times.

A similar procedure is followed for coupling of nucleic acid containing thio residues to mercurated solid support (prepared as described above in Example 4). The extent of reaction is measured by coupling $^{3}H$ nucleic acids.

c) Coupling of mercurated nucleotide to -SH containing support.

1 ml of cellulose support containing thio residues is washed with 3 ml of buffer containing Tris (50 mM) and sodium chloride (50 mM). The pH of the solution is adjusted to 7.5. 1 ml of a solution of mercurated polynucleotide (0.1 mM) is added to the cellulose suspension. The mixture is subsequently centrifuged for 30 minutes at 2000 g and the supernatant decanted. The pellet is resuspended in 1 ml buffer of Tris (50 mM) and sodium chloride (50 mM). The wash is repeated 3 times. The extent of the coupling reaction is determined by the specific release of bound nucleic acids by treatment of the suspension (a part) with 0.1 M mercaptoethanol.

The separation probes so produced, comprising a solid support having a nucleic acid moiety bonded thereto specifically can be used in the same way as immobilized nucleic acid probes heretofore available, e.g. as in assaying for sickle cell anemia genes as described in European Patent Application No. 84 107 248.1. However, because of the flexibility of the nucleic acid moiety and its greater freedom to hybridize, analyses will be faster and more accurate.

It is understood that the specification and examples are illustrative but not limitative of the present invention and that other embodiments within the spirit and scope of the invention will suggest themselves to those skilled in the art.

What is claimed is:

1. An immobilized nucleic acid containing probe characterized in that the nucleic acid is attached to a solid support by a single chemical linkage.

2. A probe according to claim 1, characterized in that the chemical linkage to the solid support is a aminoalkyl moiety.

3. A probe according to claim 1, characterized in that the chemical linkage to the solid support is a Hg-S or a S-Hg moiety.

4. A probe according to claim 1, characterized in that the chemical linkage to the solid support is a -S-S- moiety.

5. A probe according to claims 1 - 4, characterized in that the nucleic acid is a ribonucleic acid or a deoxyribonucleic acid or an oligonucleotide.

6. A probe having a nucleic acid linked thereto of the formula

nucleic acid-CH=N-solid support.

7. A nucleic acid-containing material of the formula

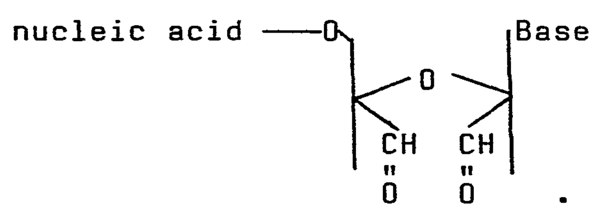

8. A nucleic acid-nucleotide unit including a purine or pyrimidine containing a Hg or -SH moiety.

9. A process for producing a probe immobilized to a solid support, characterized in that a ribonucleic acid or a oligoribonucleotide or a deoxyribonucleic acid or a deoxyoligoribonucleotide, said deoxyribonucleic acid or deoxyoligoribonucleotide being bound to a substituted sugar moiety, containing vicinale hydroxy-groups, is reacted with periodate thereby to form a nucleic acid having a pendent aldehyde group, reacting the product with a solid support having a pendent primary amino group thereby to form a Schiff's base of the formula

nucleic acid -CH=N-solid support

and reducing said Schiff's base thereby to form the desired probe.

10. A process for producing a probe immobilized to a solid support, characterized in that a ribonucleic acid or a deoxyribonucleic acid or an oligonucleotide is attached to a modified ribonucleotide or deoxynucleotide carrying on its purine or pyrimide ring a (a) Hg or (b) SH radical and reacting the coupled nucleic acid-modified nucleotide with a solid support having a penden group, in case of (a) a SH and in case of (b) a SH or Hg thereby to form the desired probe, linked to the said solid support with a Hg-S or S-Hg or -S-S- moiety.

11. A method for determining whether a ribonuclei acid or a deoxynucleic acid or an oligonucleotide contai ned in a liquid test sample includes a particular nucle: acid sequence wherein the liquid sample is contacted

0152886

with a separation probe comprising a nucleic acid immobilized to a solid support by a single chemical linkage and the separation probe and/or the remaining liquid sample is subjected to analysis to ascertain any change in its composition.

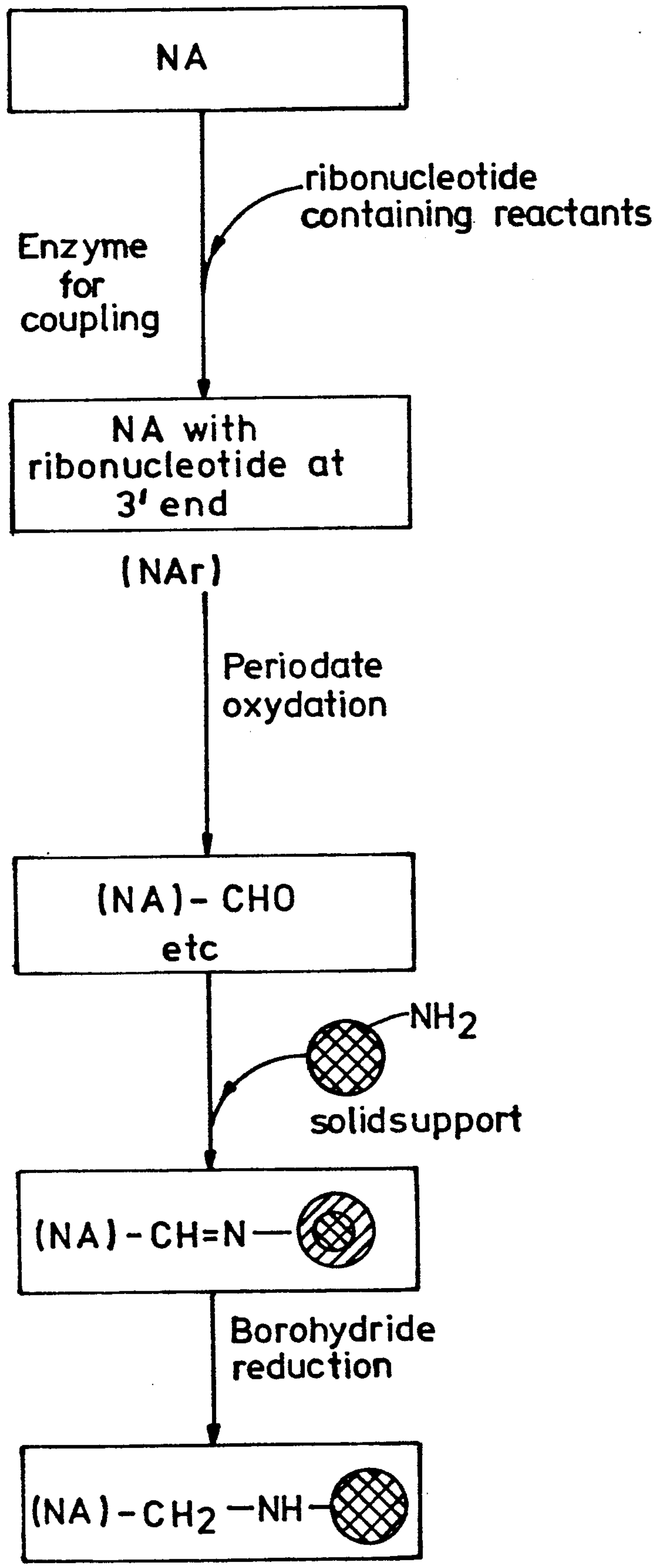
NA
ribonucleotide containing reactants
Enzyme for coupling
NA with ribonucleotide at 3' end
(NAr)
Periodate oxydation
(NA)- CHO etc
NH2
solidsupport
(NA)-CH=N—
Borohydride reduction
(NA)-CH2 —NH—

FIG. 1

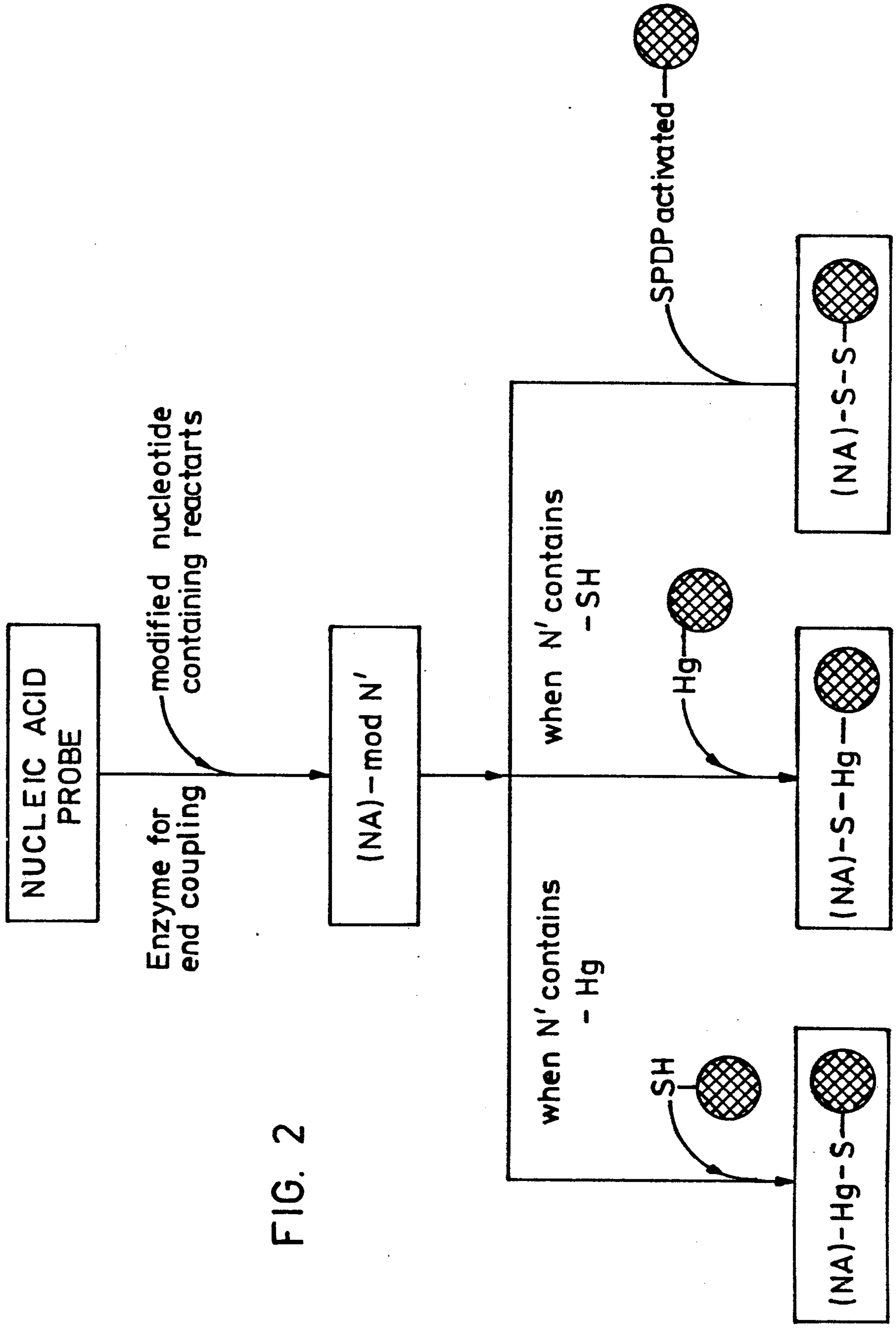
NUCLEIC ACID PROBE
Enzyme for end coupling
modified nucleotide containing reactarts
(NA)-mod N'
when N' contains -Hg
when N' contains -SH
SH
Hg
SPDP activated
(NA)-Hg-S-
(NA)-S-Hg-
(NA)-S-S-

FIG. 2